# EUROPEAN PATENT SPECIFICATION

(11) **EP 1 201 736 B1**
(45) Date of publication and mention of the grant of the patent: **27.04.2005**
(21) Application number: 00938838.0
(22) Date of filing: 27.06.2000
(51) Int. Cl.: C11B 1/00, A61K 7/00

(54) **COMPOSITIONS OF INTERNAL LIPID EXTRACT OF WOOL AND USE THEREOF IN THE PREPARATION OF PRODUCTS FOR SKIN CARE AND TREATMENT**
ZUSAMMENSETZUNGEN EINES EXTRAKTES AUS INNEREM FETT AUS WOLLE UND DESSEN VERWENDUNG ZUR HERSTELLUNG VON PRODUKTEN ZUR HAUTPFLEGE UND -BEHANDLUNG
COMPOSITIONS COMPRENANT UN EXTRAIT DE LIPIDES INTERNES DE LA LAINE ET UTILISATION DE CET EXTRAIT DANS LA PREPARATION DE PRODUITS POUR LE TRAITEMENT ET LE SOIN DE LA PEAU

(30) Priority: 09.07.1999 ES 9901541
(43) Date of publication of application: 02.05.2002
(73) Proprietor: CONSEJO SUPERIOR DE INVESTIGACIONES CIENTIFICAS, 28006 Madrid (ES)
(72) Inventor: CODERCH NEGRA, M , Luisa, Insto. de Investi., E-08034 Barcelona (ES); FONOLLOSA ARGEMI, Jorge, Insto. de Investi., E-08034 Barcelona (ES); PERA LOPEZ, Montserrat de, Insto. de Investi., E-08034 Barcelona (ES); MAZA RIBERA, Alfonso de la, Insto. de Invest., E-08034 Barcelona (ES); PARRA JUEZ, José Luis, Insto. de Investi., E-08034 Barcelona (ES); MARTI GELABERT, Meritxell, Insto. de Investi., E-08034 Barcelona (ES)
(74) Representative: Adams, Harvey Vaughan John
(86) International application number: PCT/ES2000/000227
(87) International publication number: WO 2001/004244

(56) References cited:
- WO-A1-98/46558
- FR-A1- 2 753 200
- US-A- 3 619 116
- US-A- 5 830 481
- L. CODERCH, A. DE LA MAZA ET AL.: 'Chromatographic characterization of international polar lipds from wool' JACOS vol. 72, no. 6, 1995, pages 715 - 720, XP000986281
- L. CODERCH, A. DE LA MAZA ET AL.: 'Physicochemical characteristics of liposomes formed with international wool lipids' JACOS vol. 73, no. 12, 1996, pages 1713 - 1718, XP000986282
- L. CODERCH, M. DEPERA ET AL.: 'The effect of liposomes, on skin barrier structure skin pharmacol. appl. skin' PHYSIOL. vol. 12, 1999, pages 235 - 246, XP001007438

## Description

### Field of the art

The present invention relates to the use of lipid extracts internal to wool, rich in ceramides, in the preparation of pharmaceutical products for the treatment of skin and in cosmetic products for the care of the skin.

### Previous state of the art

The term ceramide is assigned to a series of natural lipid structures, such as those described by Downing D.T. et al in The Journal of Investigative Dermatology 84:410-412 (1985). Said ceramides are fatty amides of sphingosine or phytosphingosine and are usually grouped into six types, as indicated in the aforementioned publication of Downing et al

The ceramides localised in the stratum corneum of the skin form a substantial part of the lipid barrier of human skin, a barrier that is responsible for skin protection against aggressions from outside medium and for maintaining the necessary hydration balance for good skin preservation.

The use of ceramides is well known, both of natural and synthetic origin, in different products for the treatment and care of the skin. Here, it is mentioned, by way of example only, the description of the US patent US-A-5830481.

Some extracts of natural products have been described rich that are in ceramides, and their occasion use in cosmetics. Thus, in patent application FR-A-2753200 an extract of lecithin from soy is described, in patent application WO92/13543 an extract from silk worm larvae is described, and in patent applications FR-A-2739853 and WO-A-98/46558 extracts from morera leaves are described.

The need remains, therefore, to find new natural extracts rich in ceramides from accessible and cheap materials, which allow cosmetic and pharmaceutical products to be obtained that show improved properties with respect to care and treatment of the skin, above all in terms of improving the degree of hydration. In addition, given their natural origin, they are better accepted than products of synthetic origin.

On the other hand, although the use of lanolin in cosmetic and pharmaceutical products for skincare is well known, it is worthwhile bearing in mind that lanolin is the set of lipids that are found on the surface of the wool fibres, and not inside, because said lipids are secreted by the skin of the sheep and are deposited on the surface of the fibres by means of simple contact. There is no relationship between these and the lipids of internal structures of wool fibres, which, as far as the authors of the present invention are aware, to date, have not been described for use in cosmetic and/or pharmaceutical products for skincare.

In the publication L. Coderch et al. *J. Am Oil Chem. Soc.* 72:715-720 (1995) describe the lipids of internal structures of wool fibres, as well as a method of extraction to obtain them, and the same authors, in the publication L. Coderch et al. *J. Am. Oil Chem. Soc.* 73:1713-1718 (1996), describe liposomal structures formed by said lipids and a method for obtaining them.

### Object of the invention

The object of the present invention is the use of extracts of lipids from the inner parts of wool, with a relatively high content of ceramidcs, in the preparation pharmaceutical products for the treatment of skin and in cosmetic products for skincare. The pharmaceutical compositions that contain the aforementioned extracts and procedures for the production thereof also form part of said object.

### Description of the invention

The extracts of lipids from the inner parts of wool suitable for the object of the present invention, to which hereinafter will also be referred to as IWL (Internal Wool Lipids), are characterised in that they contain at least 15% by weight, preferably more than 25% by weight, of natural ceramides of wool, along with other lipid components such as cholesterol, fatty acids and other lesser components.

Said extracts arc obtained from wool fibres from sheep of different breeds and varieties, such as Spanish merino. Australian merino. South African merino, New Zealand merino, etc., after submitting said fibres to a process of surface lipid elimination using well known techniques such as washing with detergents or as is described, for example, in the United States patent US-A-3619116. Thus, the extracts object of the invention are substantially free of lanolin.

Once treated to eliminate surface lipids, the wool fibres are submitted to a Soxhlet type extraction with an azeotropic mixture of chloroform and methanol, as described in the publication L. Coderch et al. *J. Am Oil Chem. Soc.* 72:715-720 (1995), or by means of a supercritical extraction with CO₂, well known to those versed in the art, as will be explained in more detail in the section of examples.

Either of the aforementioned methods for extraction has the advantage that the physical structure of the wool fibres is not substantially affected, maintaining its characteristics of resistance and elasticity. The extracted wool therefore retains is usefulness for subsequent industrial processing in, for example, the manufacture of textiles.

The elimination of solvents of the solutions obtained during the process of extraction leads to extracts of internal wool lipids being obtained, suitable for the object of the present invention, which can be used indistinctly in the form of oily solutions in physiologically acceptable lipophylic solvents, or in the form of liposomal structures such as those described in the publication L. Coderch et al. *J. Am. Oil Chem. Soc.* 73:1713-1718 (1996). The liposomal forms are preferred due to their stability, ease of handling and formulation, and because of their excellent skin absorption properties.

Analysis of the extracts by means of thin layer chromatography (TLC) coupled to flame ionisation detector (FID) system shows a ceramide content of greater than 15% by weight with respect to the total lipids extracted, preferably greater than 25% by weight, and with variable free fatty acid and cholesterol contents, lying, for each one of the two aforementioned components, between approximately 5% by weight and approximately 30% by weight with respect to the total of lipids extracted, in addition to other minority components.

In general, the technique of supercritical extraction with CO₂ provides extracts with a somewhat greater ceramide content using the Soxhlet extraction technique but, on the other hand, the yield of the extraction is somewhat smaller. Both types of extract are useful for the purposes of the present invention and, as a result, should be considered as lying within the scope thereof.

The tests of application of the IWLs to human skin, which will be explained in more detail in the section of examples, show that said extracts show a sharp reduction in transepidermal water loss (TEWL) and an improvement in the ability to hydrate the skin, above all in conditions of chemical or mechanical attack, with values clearly more favourable than those obtained with comparative samples of compositions of commercially available ceramides that imitate the nature of the lipids of the stratum corneum.

Thus, the IWLs object of the present invention can be formulated advantageously in different proportions, as essential components in pharmaceutical and/or cosmetic compositions for the care and treatment of human skin.

The aforementioned pharmaceutical and/or cosmetic compositions can be any of the types known by one versed in the art for topical application, such as pomades, ointments, creams, emulsions, solutions, tinctures, etc, which may be prepared by association of IWLs with physiologically acceptable excipients and other components of a suitable finish, using techniques well known to those skilled in the art such as mixing, dissolving, the types of composition and additives and auxiliary components described in the US patent US-A-5830481.

The compositions thus obtained show an excellent capacity for reinforcing the protecting lipid barrier of human skin and measurably improve the degree of hydration of said barrier.

The examples that are given below are for providing an expert in the art with a sufficiently clear and complete explanation of the present invention, but they should not be considered as limitations to the essential aspects of the object thereof, as has been presented in previous sections of this specification.

### Examples

### Example 1:- Prior conditioning of the wool fibres

The wool is obtained from living sheep by physical shearing and then the surface fats, also called lanolin, are removed by means of an industrial washing, generally in washing sets consisting of 6 troughs, using a mixture of sodium carbonate and non-ionic surfactant oxyethylenate with 8 to 9 moles of ethylene oxide as detergent. Once the wool has been delanolinised, it is submitted to a treatment of combing the fibres and elimination of mechanical impurities and, then, to soaking in distilled water for a short period of time, in order to eliminate part of the dust and other fine particles contained therein. Finally, the wool fibres are dried at room temperature and humidity, finishing the conditioning thereof with a period of 24 hours at 20° C and a RH of 60%.

### Example 2:- Extraction of internal lipids using Soxhlet

12 g of conditioned wool obtained from example 1 are weighed and introduced into a Soxhlet fitted with a cellulous cartridge to prevent the passage of any foreign particles still remaining in the wool. The extractor solvent is a mixture chloroform/methanol (79:21) with a bath ratio of 1/30, which implies a solvent volume of 360 mL. The extraction is performed at 72° C for 5 hours, a time period equivalent to six cycles of the Soxhlet. The distillate with the lipid extract is evaporated using a rotavapor apparatus, made up to 10 mL in a solution of chloroform/methanol (2:1) and stored at refrigeration temperature (4° C). Then, 1 mL of solution is evaporated to determine, using a gravimetric method, the amount extracted.

By means of the procedure presented above five varieties of merino wool are extracted which provide the following extraction yields, in which the percentage is expressed by weight of IWL obtained with respect to the initial weight of the wool fibre extracted:

| | |
|---|---|
| Spanish | 0.947 % |
| South African | 0.916% |
| New Zealand | 1.037 % |
| Australian | 1.012 % |
| Russian | 1.415 % |

For a quantitative analysis of the IWLs obtained a thin layer chromatography (TLC) technique is used coupled to a flame ionisation detector (FID) in the automatic apparatus latroscan® (Latron Lab. Inc. Tokyo, Japan). The lipid extracts are deposited directly (1.6 µL) with an automatic depositor (Sample spotter SES 3202/IS-01, Germany) on silica columns 10 cm long (Silica Gel SIII Chromarods) and are eluted vertically with three mixtures of solvents in order of decreasing polarity: 1) twice with chloroform/methanol/water (57:12:0.6) up to 2.5 cm; 2) Hexane/Diethyl ether/Formic acetate (50:20:0.3) up to 8 cm; and 3) Hexane/Benzene (35:35) up to 10 cm. After each elution, the solvent is removed by heating at 60° C followed by cooling in a chamber at room temperature and dryness. The same procedure is applied to the standards: Palmitic acetate, cholesterol and ceramide III to thus determine their calibration curves and enabling subsequent quantification of each component. The standards are acquired from Sigma Co (St. Louis, M.O.) in analytic grade and stored in chloroform/methanol under refrigeration until the moment in which they are used.

The results obtained with the five samples of wool extracted are presented in table I, in which said results are expressed as percentages by weight with respect to total weight of IWL extract.

**Table I:-**

| Chemical composition of the IWLs obtained by means of extraction procedure with Soxhlet. | | | | |
|---|---|---|---|---|
| Variety of merino wool | Free fatty acids (%) | Cholesterol (%) | Ceramides (%) | Rest (*) (%) |
| Spanish | 20.09 | 21.25 | 31.88 | 26.79 |
| South African | 22.44 | 24.74 | 29.30 | 23.52 |
| New Zealand | 32.57 | 17.70 | 27.65 | 22.08 |
| Australian | 23.39 | 25.28 | 25.98 | 25.35 |
| Russian | 13.30 | 12.71 | 34.89 | 39.10 |

| | | | | |
|---|---|---|---|---|
| (*) Remaining lipid components not quantified, which are found in lower proportions, such as cholesterol esters, mono-, di- and triglycerides, derivatives of oxidated cholesterol, cerebrosides, cholesterol sulphate, etc. | | | | |

### Example 3:- Supercritical extraction of internal lipids using CO₂ (SFE)

The apparatus used to perform the supercritical extraction has a double syringe pump (SFC-3000; Fisons, Milan, Italy) that distributes the CO₂ (SFE grade 99.998%, Praxir España, Barcelona, Spain) and the modifier of polarity (Methanol in analytical grade, Merck, Darmstad, Germany), in the desired proportions. The function of the modifier is to increase the capacity of extraction and dissolving of CO₂. The flow of liquid is adjusted by means of a regulating valve (Hoke Inc, Creskill, NJ, USA) to 1.5-2.0 mL.min⁻¹, measured from the LCD of the pump, keeping the temperature at 100° C.

4.8 g of wool conditioned in accordance with the procedure of example 1 are introduced into the cylinder where the extraction takes place in supercritical conditions for CO₂, these being 340 atmospheres, 20 min, at a temperature of 100" C and 10% methanol. The total volume of liquid used for the extraction is 4-5 times the volume of the cell. The extract is collected in a 15-mL vial sealed beforehand with septum (Supelco, Bellefonte PA, USA) which has an outlet to eliminate decompressed CO₂. The extract is concentrated until dry under nitrogen flow, or by heating to 60° C, in order to eliminate excess solvent. The IWL extracted is quantified using a gravimetric method and resuspended in chloroform/methanol 2:1 (1 mL), keeping under refrigeration.

Following the extraction procedure described above, two varieties of merino wool are extracted which provide the following extraction yields, in which the percentage is expressed by weight of IWL extract with respect to the initial weight of the wool fibre extracted.

| | |
|---|---|
| Spanish | 0.290 % |
| New Zealand | 0,355 % |

For quantitative analysis of the IWL obtained the chromatographic technique already described in example 2 is used, and the results obtained with the two samples of wool extracted are presented in table II, in which said results are expressed as percentages by weight with respect to total weight of IWL extract.

**Table II.-**

| Chemical composition of the IWLs obtained by means of supercritical extraction with CO₂ (SFE) | | | | |
|---|---|---|---|---|
| Variety of merino wool | Free fatty acids (%) | Cholesterol (%) | Ceramides (%) | Rest (*) (%) |
| Spanish | 17.75 | 7.41 | 45.29 | 29.55 |
| New Zealand | 26.02 | 7.75 | 29.73 | 36.50 |

| | | | | |
|---|---|---|---|---|
| (*) Remaining lipid components not quantified, which are found in lower proportions, such as cholesterol esters, mono-, di- and triglycerides, derivatives of oxidated cholesterol, cerebrosides, cholesterol sulphate, etc. | | | | |

As can be seen from comparison of the data obtained in examples 2 and 3, the method of extraction using the Soxhlet provides much better extraction yields, but the SFE method has a smaller affinity for the extraction of cholesterol and a somewhat greater yield with respect to the extraction of the ceramides.

### Example 4:- Mechanical characteristics of the treated wool fibres

The wool fibres treated in accordance with the procedures described in examples 2 and 3 are conditioned for 48 hours at 20° C and 60 % R.H. A representative stream of the sample is chosen, immobilized, combed, and weighed and then submitted to the mechanical tests of resistance outlined in the IWTO guideline (E)-5-73 (E) appendix B, recognised in the wool textile industrial sector. The test are carried out ten times, always keeping a grip separation of 5 mm and a deformation gradient of 60 %/min (3 mm/min) using an Instron 5500 strength tester coupled to a computer.

In table III, the results are shown regarding the toughness expressed in cN/tex, in other words, in centiNewtons (cN) per mg mass of fibre for each metre of fibre (tex), and with regard to deformation, expressed in percentage, compared to a control that corresponds to wool fibres that have not been submitted to the extraction treatment.

**Table III:-**

| Mechanical characteristics of the wool fibres treated. | | | |
|---|---|---|---|
| Merino variety | Treatment | Roughness cN/tex (% coef. of variance) | Deformation % (% coef. of variance |
| Spanish | Not treated | 8.91 (11.98) | 84.32 (3.75) |
| | Example 2 | 7.11 (10.52) | 119.62 (2.85) |
| | Example 3 | 7.89 (26.35) | 43.83 (12.58) |
| South Atrican | Not treated | 9.23 (7.00) | 126.92 (6.28) |
| | Example 2 | 9.63 (9.41) | 127.82 (6.74) |
| Australian | Not treated | 10.34 (10.71) | 113.52 (16.28) |
| | Example 2 | 10.08 (9.68) | 136.92 (11.05) |
| New Zealand | Not treated | 13.64 (7.3) | 64.49 (5.5) |
| | Example 2 | 12.18 (12.4) | 62.46 (11.5) |
| | Example 3 | 9.47 (13.17) | 55.63 (16.91) |

With regard to the toughness parameter, no important change is observed due to treatment of extraction of the IWLs, either by Soxhlet or by SFE, nor is there a clear tendency to increase or decrease the value. With regard to deformation, the treatment by Soxhlet leads to an increase in these values which could be important in some stages of industrial processing of this material, such as those of combing or spinning. This treatment does not negatively affect the properties of the wool fibres, and the textile produced can be used for normal purposes. With regard to the SFE treatment, the deformation values observed are somewhat smaller than those for the untreated samples, although the use of the textile is not invalidated by this.

As a result, the procedures for extraction of IWLs from wool described in examples 2 and 3 does not significantly modify the mechanical properties of the wool.

### Example 5:- Procedure for obtaining the liposomal forms

An IWL extract dissolved in chloroform/methanol 2:1 (v/v) from either example 2 or 3 is evaporated to dryness under a nitrogen atmosphere, and the film formed on the walls of the recipient is resuspended with a suitable volume of NaCl solution at 0.9% by weight to obtain a suspension of lipid concentration of 10 mg/ml. The formation of multi-lamina liposomes is carried out be sonicating the sample at 65° C, which is a temperature greater than that of phase transition of the implicated lipids, for 15 minutes in a Labsonic® 1510 sonicator (B. Braum, Germany).

After leaving to stand for 24 hours at room temperature, uni-lamina liposomes of a defined size are obtained by extruding the multi-lamina liposome suspension three times, at 65° C, through filters with a pore size of 800 x 400 x 200 nm (Millipore®, Ireland), providing a suspension of liposomes whose size can be checked to be approximately 200 nm by means of the technique of light scattering, using a Autosizer® photocorrelation IIc spectrometer (Malvern, UK).

### Example 6:- Effects of IWL extracts on healthy skin, in the short and long term

The aim of this trial is to evaluate the effect of topical application of liposomes formed from internal wool lipids on the barrier function and hydration of healthy skin, and compare this with the effect produced by the liposomes obtained from a lipid composition that simulates that of the human stratum corneum (SC) itself.

The evaluation of these properties of the skin is performed using the measures made with the Tewameter® TM 210 apparatus and the Corneometer® CM 825 apparatus, both from Courage & Khazaka (Germany). The Tewameter apparatus measures the transepidermal water loss (TEWL), which is an indicator of the state of the barrier function of the skin. High values of TEWL correspond to a low barrier function, in other words, to a skin less protected against external aggression and with lower capacity to limit the entrance of foreign substances into the body and the release of water to the environment. The Corneometer apparatus bases its measurement on the capacitance of the skin, in arbitrary units. The capacitance is directly related to the amount of water present in the skin and, therefore, the apparatus is effective at determining hydration of the skin.

The trials were carried out in six volunteers with healthy skin, one man and five women, with ages lying between 24 and 41 years, using the following preparations:
- IWL liposomes: This consists of a liposome suspension at 2.5% by weight, obtained in accordance with that described in example 5, using the IWL extract from Spanish merino wool obtained in example 2.
- SC liposomes: This consists of a liposome suspension at 2.5% by weight, obtained in accordance with that described in example 5, using a lipid composition that imitates the stratum corneum of human skin itself, prepared beforehand by mixing 40 parts by weight of ceramide III supplied by Cosmoferm (Holland), 25 parts by weight of palmitic acid, 25 parts by weight of cholesterol and 10 parts by weight of cholesterol sulphate.
- Placebo: This consists of an aqueous solution of NaCl at 0.9%, similar to that used to prepare the aforementioned liposome suspensions.

After acclimatising the volunteers for 15 minutes to a room with the conditions held at 20° C and 60% R.H., two square areas of 2 cm are marked out with adhesive cells to one side of the central area of the forearm. To one of these no application will be made (control) and to the other placebo will be applied. Two equal areas are marked out on the other forearm, where IWL liposomes and SC liposomes arc applied.

Baseline measurements are performed of the TEWL and the capacitance, then 10 µl of the corresponding preparation are applied, measuring the parameters once again after two hours. Once 24 hours have passed (day 1), the parameters will be measured once again and 10 µl of preparation applied once more. This procedure is repeated for the following three days (days 2, 3 and 4). Finally, after 3 days from the day of the last application (day 7) the parameters are measured once more.

The results of the trial, double normalised with respect to the baseline value and the values obtained for the control, are shown in table IV.

**Table IV:-**

| Effects of IWL liposomes on healthy skin | | | | | | |
|---|---|---|---|---|---|---|
| | Variation (base 100) of TEWL | | | Variation (base 100) of TEWL capacitance | | |
| Time | Placebo | IWL liposomes | SC liposomes | Placebo | IWL liposomes | SC liposomes |
| 2 hour | 101.6 | 90.1 | 82.3 | 89.5 | 93.0 | 85.1 |
| 1 day | 103.7 | 91.6 | 92.3 | 95.9 | 99.9 | 100.2 |
| 2 days | 105.3 | 98.2 | 101.4 | 96.3 | 103.8 | 97.0 |
| 3 days | 99.1 | 95.8 | 98.3 | 104.8 | 107.0 | 106.2 |
| 4 days | 104.2 | 96.3 | 93.2 | 103.3 | 106.4 | 104.8 |
| 7 days | 101.9 | 79.6 | 85.5 | 98.2 | 107.5 | 100.4 |

It is clearly observed that the liposome preparations improve the barrier effect of the skin during the course of time (reduction of TEWL), although the IWL liposome preparation, object of the present invention, appears more effective over long periods of time.

With regard to hydration of the skin (increase of capacitance), only the preparation of IWL object of the invention produces an increase therein which is maintained for the seven days of the trial.

### Example 7:- Short-term effects of the IWL extracts on skin attacked chemically

In the same fashion as in example 6, with the same group of volunteers, the trials are conducted with the variations that are presented below.

The areas of skin to be tested are immersed for 15 minutes in a 1% solution by weight of sodium laurylsulphate heated to 35° C. After this, the skin is washed with abundant amounts of distilled water and the treated area dried with absorbent paper.

The preparation of IWL liposomes applied consists of a liposome suspension at 1% by weight, obtained in accordance with that described in example 5, using the IWL extract from New Zealand merino wool obtained in example 2. The SC liposome preparation is the same as that applied in example 6, but diluted to a concentration of 1% by weight.

The baseline measurement of the TEWL and capacitance parameters is performed before applying the treatment with the surfactant solution, and the measurement which is considered at time 0 is performed after leaving standing for an hour after finishing the aforementioned treatment. Then, 10 µl of preparation corresponding to each marked area of the skin is applied. The aforementioned measurements are made after 30 minutes, 1, 2 and 3 hours.

In the present case, measurements of the TEWL parameter do not permit conclusions to be drawn, as the measurement at time 0 is practically identical (10.7 g/m²/h) to that obtained at baseline (10.8 g/m²/h), indicating that the healthy skin has recovered on its own from the degradation of the barrier effect induced in the first instance by the surfactant in less than an hour.

With regard to the capacitance parameter (hydration), the results obtained are shown in table V, expressed as % variation with respect to the results obtained for the control area with no application, taking into account that the values for each series have been divided by the value obtained at time 0 for said series, with the aim of balancing the initial conditions of the trial.

**Table V.**

| Effects of IWL liposomes on skin attacked chemically | | | |
|---|---|---|---|
| | Variation in % of capacitance | | |
| Time (hours) | Placebo | IWL Liposomes | SC liposomes |
| 0 | 0.0 | 0.0 | 0.0 |
| 0.5 | 17.8 | 23.1 | 17.3 |
| 1 | 15.2 | 20.6 | 14.7 |
| 2 | 16.5 | 20.3 | 19.6 |
| 3 | 16.1 | 21.9 | 15.4 |

Although the three preparations improve the hydration of the skin, which indicates that the saline solution itself (placebo) shows beneficial effects, the IWL liposomes show an increase in the hydration of the skin attacked chemically beforehand by the anionic surfactant used that is clearly better than the other two preparations tested.

### Example 8 Short-term effects of the IWL extracts on mechanically attacked skin

In the same fashion as in example 6, with the same group of volunteers, trials are performed introducing the variations that are described below.

The areas of the skin to be tested are submitted to 15 operations of sticking and removal of strips of Scotch Magic® 810 adhesive tape. The technique, known as stripping, allows layers of stratum corneum of skin to be removed.

Before applying the technique of stripping, the baseline parameters of TEWL and capacitance are determined, and immediately after performing the 15 stripping actions, these parameters are measured for a time 0. Next, the samples are applied as described in example 7.

The results obtained are shown in table VI, expressed, as in example 7, as % variation with respect to those obtained for the control area with no application, taking into account that the values of each time 0 for each series have been divided in order to balance the conditions of the trial.

**Table VI:-**

| Effects of IWL liposomes on mechanically attacked skin | | | | | | |
|---|---|---|---|---|---|---|
| | Variation in % of TEWL | | | Variation in % of capacitance | | |
| lime (hours) | Placebo | IWL liposomes | SC liposomes | Placebo | IWL liposomes | SC liposomes |
| 0 | 0.0 | 0.0 | 0.0 | 0.0 | 0.0 | 0.0 |
| 0.5 | 6.1 | -7.8 | 10.0 | -2.5 | 8.0 | 7.6 |
| 1 | 5.6 | -9.1 | 7.6 | 0.8 | 9.1 | 5.2 |
| 2 | 7.9 | -3.7 | 8.2 | 4.4 | 9.6 | 7.1 |
| 3 | 5.5 | -6.0 | 17.6 | 5.7 | 8.9 | 6.5 |

The preparation of IWL liposomes is the only one of those tested that showed a clear capacity for regeneration of the barrier effect (lower TEWL) in mechanically attacked skin. Of note is that it is much better than the SC liposomes, consisting of a lipid composition similar to that found in the stratum corneum of the skin itself.

Once again, although the skin hydration (capacitance) is increased by the three preparations tested, the preparation of IWL liposomes leads to a greater improvement in the hydration of the skin than the other two preparations.

## Claims

1. Use of an internal wool lipid extract that contains at least 15% by weight of ceramides in a cosmetic composition for the care of skin.

2. Use of an internal wool lipid extract that contains at least 15% by weight of ceramides in the manufacture of a pharmaceutical composition for the treatment of the skin.

3. Use, according to claim I or 2, **characterised in that** the internal wool lipid extract contains at least 25% by weight of ceramides.

4. Use, according to any of the previous claims, **characterised in that** the internal wool lipid extract also contains free fatty acids and cholesterol in proportions, mutually independent, lying between 5% by weight and 30% by weight with respect to the total weight of the extract.

5. Use, according to any of the previous claims, **characterised in that** the internal wool lipid extract is employed in the form of liposomes.

6. Use, according to claim 5, **characterised in that** said liposomes are found in aqueous suspension of a solution of sodium chloride.

7. A pharmaceutical composition for the treatment of skin disorders **characterised in that** it comprises an extract of internal wool lipids that contain at least 15% by weight of ceramides.

8. A composition, according to claim 7, **characterised in that** the extract of internal wool lipids contains at least 25% by weight of ceramides.

9. A composition, according to either claim 7 or 8, **characterised in that** the extracts of internal wool lipids also contain free fatty acids and cholesterol in proportions, mutually independent, lying between 5% by weight and 30% by weight with respect to the total weight of the extract.

10. A composition, according to any of claims 7 to 9, **characterised in that** the extract of internal wool lipids are in the form of liposomes.

11. A composition, according to claim 10, **characterised in that** said liposomes are found in an aqueous suspension of a solution of sodium chloride.

12. A procedure for obtaining to pharmaceutical composition of claims 7 to 11, **characterised in that** it consists of associating the ceramide extracts of claims 1 to 6 with physiologically acceptable excipients.

13. An extract of internal wool lipids that contains at least 15% by weight of ceramides, for the treatment of skin disorders.

14. An extract, according to claim 13, **characterised in that** it contains at least 25% by weight of ceramides.

15. An extract, according to either of claims 13 or 14, **characterised in that**, in addition, it contains free fatty acids and cholesterol in proportions, mutually independent, of between 5% by weight and 30% by weight with respect to the total weight of the extract.

16. An extract, according to any of claims 13 to 15, **characterised in that** it is found in the form of liposomes.

17. An extract, according to claim 16, **characterised in that** said liposomes are found in an aqueous suspension of a solution of sodium chloride.

## Patentansprüche

1. Verwendung eines Extrakts interner Wolllipide, der mindestens 15 Gew.-% Ceramide enthält, in einer kosmetischen Zusammensetzung fiir die Pflege der Haut.

2. Verwendung eines Extrakts interner Wolllipide, der mindestens 15 Gew.-% Ceramide enthält, in der Herstellung einer pharmazeutischen Zusammensetzung zur Behandlung der Haut.

3. Verwendung nach Anspruch 1 oder 2, **dadurch gekennzeichnet, dass** der Extrakt der internen Wolllipide mindestens 25 Gew.-% Ceramide enthält.

4. Verwendung nach einem der vorhergehenden Ansprüche, **dadurch gekennzeichnet, dass** der Extrakt der internen Wolllipide des Weiteren freie Fettsäuren und Cholesterol in gegenseitig unabhängigen Anteilen enthält, die zwischen 5 Gew.% und 30 Gew.-%, bezogen auf das Gesamtgewicht des Extraktes, liegen.

5. Verwendung nach einem der vorhergehenden Ansprüche, **dadurch gekennzeichnet, dass** der Extrakt der internen Wolllipide in der Form von Liposomen verwendet wird.

6. Verwendung nach Anspruch 5, **dadurch gekennzeichnet, dass** die Liposomen in wässriger Suspension einer Natriumchloridlösung gefunden werden.

7. Pharmazeutische Zusammensetzung zur Behandlung von Hauterkrankungen bzw. -störungen, **dadurch gekennzeichnet, dass** sie einen Extrakt interner Wolllipide umfasst, der mindestens 15 Gew.-% Ceramide enthält.

8. Zusammensetzung nach Anspruch 7, **dadurch gekennzeichnet, dass** der Extrakt der internen Wolllipide mindestens 25 Gew.-% Ceramide enthält.

9. Zusammensetzung entweder nach Anspruch 7 oder 8, **dadurch gekennzeichnet, dass** die Extrakte der internen Wolllipide des Weiteren freie Fettsäuren und Cholesterol in gegenseitig unabhängigen Anteilen enthalten, die zwischen 5 Gew.-% und 30 Gew.-%, bezogen auf das Gesamtgewicht des Extrakts, liegen.

10. Zusammensetzung nach einem der Ansprüche 7 bis 9, **dadurch gekennzeichnet, dass** der Extrakt der inneren Wolllipide in der Form von Liposomen vorliegt.

11. Zusammensetzung nach Anspruch 10, **dadurch gekennzeichnet, dass** die Liposomen in einer wässrigen Suspension einer Natriumchloridlösung gefunden werden.

12. Verfahren zum Erhalten einer pharmazeutischen Zusammensetzung der Ansprüche 7 bis 11, **dadurch gekennzeichnet, dass** es aus der Vereinigung der Ceramidextrakte der Ansprüche 1 bis 6 mit physiologisch annehmbaren Hilfsstoffen besteht.

13. Extrakt interner Wolllipide, der mindestens 15 Gew.-% Ceramide enthält, zur Behandlung von Hauterkrankungen.

14. Extrakt nach Anspruch 13, **dadurch gekennzeichnet, dass** er mindestens 25 Gew.-% Ceramide enthält.

15. Extrakt entweder nach Anspruch 13 oder 14, **dadurch gekennzeichnet, dass** er zusätzlich freie Fettsäuren und Cholesterol in gegenseitig unabhängigen Anteilen von zwischen 5 Gew.-% und 30 Gew.-%, bezogen auf das Gesamtgewicht des Extrakts, enthält.

16. Extrakt nach einem der Ansprüche 13 bis 15, **dadurch gekennzeichnet, dass** er in Form von Liposomen gefunden wird.

17. Extrakt nach Anspruch 16, **dadurch gekennzeichnet, dass** die Liposomen in einer wässrigen Suspension einer Natriumchloridlösung gefunden werden.

## Revendications

1. Utilisation d'un extrait de lipides internes de la laine qui contient au moins 15 % en poids de céramides dans une composition cosmétique pour les soins de la peau.

2. Utilisation d'un extrait de lipides internes de la laine qui contient au moins 15 % en poids de céramides dans la fabrication d'une composition pharmaceutique pour le traitement de la peau.

3. Utilisation, selon les revendications 1 ou 2, **caractérisée en ce que** l'extrait de lipides internes de la laine contient au moins 25 % en poids de céramides.

4. Utilisation, selon l'une quelconque des revendications précédentes, **caractérisée en ce que** l'extrait de lipides internes de la laine contient également des acides gras libres et du cholestérol dans des proportions, mutuellement indépendantes, se trouvant entre 5 % en poids et 30 % en poids par rapport au poids total de l'extrait.

5. Utilisation, selon l'une quelconque des revendications précédentes, **caractérisée en ce que** l'extrait de lipides internes de la laine est employé sous la forme de liposomes.

6. Utilisation, selon la revendication 5, **caractérisée en ce que** lesdits liposomes se trouvent en suspension aqueuse dans une solution de chlocure de sodium.

7. Composition pharmaceutique pour le traitement des troubles cutanés **caractérisée en ce qu'**elle comprend un extrait de lipides internes de la laine qui contient au moins 15 % en poids de céramides.

8. Composition, selon la revendication 7, **caractérisée en ce que** l'extrait de lipides internes de la laine contient au moins 25 % en poids de céramides.

9. Composition, selon l'une ou l'autre des revendications 7 ou 8, **caractérisée en ce que** l'extrait de lipides internes de la laine contient également des acides gras libres et du cholestérol dans des proportions, mutuellement indépendantes, se trouvant entre 5 % en poids et 30 % en poids par rapport au poids total de l'extrait.

10. Composition, selon l'une quelconque des revendications 7 à 9, **caractérisée en ce que** l'extrait de lipides internes de la laine est sous la forme de liposomes.

11. Composition, selon la revendication 5, **caractérisée en ce que** lesdits liposomes se trouvent en une suspension aqueuse dans une solution de chlorure de sodium.

12. Procédure pour obtenir une composition pharmaceutique selon les revendications 7 à 11, **caractérisée en ce qu'**elle consiste à associer les extraits de céramides selon les revendications 1 à 6 avec des excipients physiologiquement acceptables.

13. Extrait de lipides internes de la laine qui contient au moins 15 % en poids de cérmides, pour le traitement de troubles cutanés.

14. Extrait, selon la revendication 13, **caractérisé en ce qu'**il contient au moins 25 % en poids de céramides.

15. Extrait, selon l'une ou l'autre des revendications 13 ou 14, **caractérisée en ce que**, de plus, il contient des acides gras libres et du cholestérol dans des proportions, mutuellement indépendantes, se trouvant entre 5 % en poids et 30 % en poids par rapport au poids total de l'extrait.

16. Extrait, selon l'une quelconque des revendications 13 à 15, **caractérisée en ce qu'**il se trouve sous la forme de liposomes.

17. Extrait, selon la revendication 16, **caractérisée en ce que** lesdits liposomes se trouvent en une suspension aqueuse dans une solution de chlorure de sodium.
